# EUROPÄISCHE PATENTANMELDUNG

(11) **EP 1 002 796 A2**
(43) Veröffentlichungstag der Anmeldung: **24.05.2000**
(21) Anmeldenummer: 00250046.0
(22) Anmeldetag: 28.04.1994
(51) Int. Cl.: C07D 241/44, A61K 31/498, A61P 25/00

(54) **Neue Chinoxalindionderivate, deren Herstellung und Verwendung in Arzneimitteln**

(30) Priorität: 28.04.1993 DE 4314591; 21.12.1993 DE 4344486
(62) Teilanmeldung aus: 94914313.5
(71) Anmelder: Schering Aktiengesellschaft, 13353 Berlin (DE)
(72) Erfinder: Huth, Andreas, 12437 Berlin (DE); Turski, Lechoslav, 13505 Berlin (DE)

(57) **Zusammenfassung**

Es werden Verbindungen der Formel I beschrieben 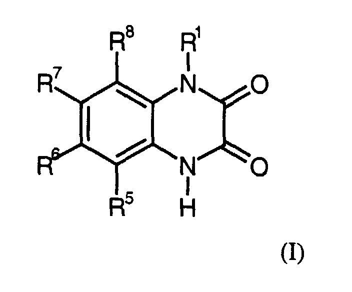 worin
R¹ R⁵, R⁶, R⁷ und R⁸ die in der Anmeldung genannten Bedeutungen haben sowie deren Herstellung und Verwendung in Arzneimitteln.

## Beschreibung

Die Erfindung betrifft Chinoxalindionderivate, deren Herstellung und Verwendung in Arzneimitteln.

Es ist bekannt, daß Chinoxalinderivate Affinität an die Quisqualat-Rezeptoren besitzen und sich auf Grund der Affinität als Arzneimittel zur Behandlung von Krankheiten des zentralen Nervensystems eignen.

Die erfindungsgemäßen Verbindungen haben die Formel I 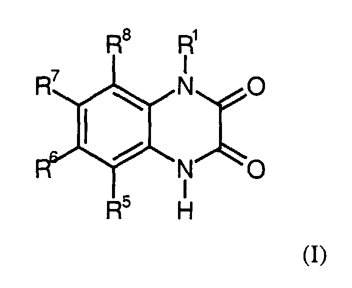 worin
R¹ -(CH₂)ₙ-CR²H-(CH₂)ₘ-Z und
R⁵, R⁶, R⁷ und R⁸ gleich oder verschieden sind und Wasserstoff C₁₋₆-Alkyl, CF₃, Nitro, Halogen, NR⁹R¹⁰, Cyano, SOₚR¹¹, SO₂NR¹²R¹³, SO₃H, SO₃C₁₋₆-Alkyl oder OR¹⁴ bedeuten,
   wobei
R² Wasserstoff oder -(CH₂)_{q}-R³,
R³ Wasserstoff, Hydroxy, C₁₋₆-Alkoxy oder NR¹⁵R¹⁶,
n, m und q jeweils 0, 1, 2 oder 3
Z POXY, OPOXY, OR¹⁷, NR¹⁸R¹⁹, NH-COR²⁰, NH-SO₂R²¹, SO₂R²², CO₂R²³ Halogen, Cyano oder Tetrazol,
R¹¹ H, C₁₋₆-Alkyl, Phenyl,
p 0,1 oder 2
R¹², R¹³, R¹⁷ und R²³ Wasserstoff oder C₁₋₄-Alkyl,
R¹⁴ H oder gegebenenfalls 1-3 fach mit Halogen substituiertes C₁₋₆-Alkyl,
R²⁰ und R²¹ C₁₋₆-Alkyl, gegebenenfalls mit Halogen substituiertes Phenyl oder Hetaryl,
R²² Hydroxy, C₁₋₆-Alkoxy oder NR²⁴R²⁵,
X und Y gleich oder verschieden sind und Hydroxy, C₁₋₆-Alkoxy, C₁₋₄-Alkyl oder NR¹⁸R¹⁹ bedeuten,
R⁹ und R¹⁰ gleich oder verschieden sind und Wasserstoff, CO-C₁₋₆-Alkyl, Phenyl oder C₁₋₆-Alkyl, das gegebenenfalls mit C₁₋₄-Alkoxy oder einer gegebenenfalls mit C₁₋₄-Alkyl mono- oder di-substituierter Aminogruppe substituiert sein kann, oder gemeinsam mit dem Stickstoffatom einen 5-7-gliedrigen gesättigten Heterocyclus bilden, der ein weiteres N-, S- oder O-Atom enthalten und substituiert sein kann oder einen ungesättigten 5-gliedrigen Heterocyclus bilden, der 1-3 N-Atome enthalten und substituiert sein kann,
R¹⁵ und R¹⁶, R¹⁸ und R¹⁹ gleich oder verschieden sind und Wasserstoff, C₁₋₄-Alkyl, Phenyl oder gemeinsam mit dem Stickstoffatom einen 5-7-gliedrigen gesättigten Heterocyclus bilden, der ein weiteres Sauerstoff-, Schwefel- oder Stickstoffatom enthalten und substituiert sein kann oder einen ungesättigten 5-gliedrigen Heterocyclus bilden, der 1-3 N-Atome enthalten und substituiert sein kann,
R²⁴ und R²⁵ gleich oder verschieden sind und Wasserstoff, C₁₋₄-Alkyl oder gemeinsam mit dem Stickstoffatom einen gesättigten 5-7-gliedrigen Heterocyclus bilden, der ein weiteres Sauerstoff-, Schwefel- oder Stickstoffatom enthalten kann, sowie deren Isomeren oder Salze,
   wobei, falls R² Wasserstoff und Z POXY oder CO₂R²³ ist,
R⁵-R⁸ nicht Wasserstoffbedeuten und
falls R² Wasserstoff, Z POXY oder CO₂R²³ und R⁵, R⁶, R⁷ oder R⁸ CF₃, NO₂, Halogen, NH₂ oder Methyl bedeuten, disubstituierte Verbindungen der Formel I vorliegen und
falls R¹ Methanphosphonsäure und R⁶ Cyano oder substituiertes Imidazol ist, können R⁵, R⁷ und R⁸ nicht gleichzeitig Wasserstoff sein und
falls R¹ Methanphosphonsäure und R⁶ CF₃ oder NO₂ und R⁷ Imidazol ist, können R⁵ und R⁸ nicht gleichzeitig Wasserstoff sein und
falls R¹ -CH₂-COOH und R⁵ und R⁸ Wasserstoff bedeuten, können R⁶ und R⁷ nicht gleichzeitig Halogen oder Methyl bedeuten.

Die Verbindungen der allgemeinen Formel I beinhalten auch die möglichen tautomeren Formen und umfassen die E- oder Z-Isomeren oder, falls ein chirales Zentrum vorhanden ist, die Razemate oder Enantiomeren.

Die Substituenten stehen bevorzugt in 6- und/oder 7-Stellung.

Unter Alkyl ist jeweils ein geradkettiger oder verzweigter Alkylrest zu verstehen wie beispielsweise Methyl, Ethyl, Propyl, Isopropyl, Butyl, Isobutyl, sek. Butyl, Pentyl, Isopentyl, Hexyl, wobei C₁₋₄-Alkylreste bevorzugt werden.

Unter Halogen ist jeweils Fluor, Chlor, Brom und Jod, insbesondere Fluor, Chlor und Brom, zu verstehen.

Bilden R⁹ und R¹⁰, R¹⁵ und R¹⁶, R¹⁸ und R¹⁹, R²⁴ und R²⁵ gemeinsam mit dem Stickstoffatom einen gesättigten Heterocyclus, so ist beispielsweise Piperidin, Pyrrolidin, Morpholin, Thiomorpholin, Hexahydroazepin oder Piperazin gemeint. Der Heterocyclus kann 1-3-fach substituiert sein mit C₁₋₄-Alkyl oder Aryl wie Phenyl. Beispielsweise seien genannt N-Methyl-piperazin, 2,6-Dimethylmorpholin, oder Phenylpiperazin.

Bilden R⁹ und R¹⁰, R¹⁵ und R¹⁶, R¹⁸ und R¹⁹, gemeinsam mit dem Stickstoffatom einen ungesättigten Heterocyclus, so seien beispielsweise Imidazol, Pyrazol, Pyrrol und Triazol genannt, die ein-bis zweifach mit Cyano, C₁₋₄-Alkyl, Phenyl oder CO₂C₁₋₆-Alkyl substituiert sein können.
Ist eine saure Funktion enthalten sind als Salze die physiologisch verträglichen Salze organischer und anorganischer Basen geeignet wie beispielsweise die gut löslichen Alkali- und Erdalkalisalze sowie N-Methyl-glukamin, Dimethyl-glukamin, Ethyl-glukamin, Lysin, 1,6-Hexadiamin, Ethanolamin, Glukosamin, Sarkosin, Serinol, Tris-hydroxy-methylamino-methan, Aminopropandiol, Sovak-Base, 1-Amino-2,3,4-butantriol.
Ist eine basische Funktion enthalten sind die physiologisch verträglichen Salze organischer und anorganischer Säuren geeignet wie HCl, H₂SO₄, Phosphorsäure, Zitronensäure, Weinsäure u.a.

Als Hetarylrest R²⁰ und R²¹ kommen 6-Ring Heteroaromaten wie Pyridin, Pyrazin, Pyrimidin und Pyridazin in Betracht.

Bevorzugt sind Verbindungen mit Z = -POXY, -OR¹⁷, -CO₂R²³, -NR¹⁸R^{19,} SO₃H oder Tetrazol, die in 5-, 6-, 7- und/oder 8-Stellung mit C₁₋₆-Alkyl, CF₃, Nitro, Halogen, SOₚR¹¹, SO₂NR¹²R¹³ oder NR⁹R¹⁰ substituiert sind.

Besonders bevorzugt sind Phosphonsäure- und Carbonsäurederivate, die ein- bis zweifach substituiert sein können. Insbesondere bevorzugt sind Phosphonsäurederivate, die in Position 5-8 zweifach substituiert sind. Bevorzugte Substituenten R⁵ - R⁸ sind insbesondere NR⁹R¹⁰ und CF₃.

Die Verbindungen der Formel I sowie deren physiologisch verträglichen Salze sind auf Grund ihrer Affinität zu den AMPA-Rezeptoren als Arzneimittel verwendbar. Auf Grund ihres Wirkprofils eignen sich die erfindungsgemäßen Verbindungen zur Behandlung von Krankheiten, die durch Hyperaktivität excitatorischer Aminosäuren wie zum Beispiel Glutamat oder Aspartat hervorgerufen werden. Da die neuen Verbindungen als Antagonisten excitatorischer Aminosäuren wirken und eine hohe spezifische Affinität zu den AMPA-Rezeptoren zeigen, indem sie den radioaktiv markierten spezifischen Agonisten (RS)α-Amino-3-hydroxy-5-methyl-4-isoxazolpropionat (AMPA) von den AMPA-Rezeptoren verdrängen, eignen sie sich insbesondere zur Behandlung von solchen Krankheiten, die über die Rezeptoren excitatorischer Aminosäuren, insbesondere den AMPA-Rezeptor, beeinflußt werden.

Erfindungsgemäß können die Verbindungen verwendet werden zur Behandlung neurologischer und psychiatrischer Störungen, die durch die Überstimulation des AMPA-Rezeptors ausgelöst werden. Zu den neurologischen Erkrankungen , die funktionell und präventiv behandelt werden können, gehören zum Beispiel neurodegenerative Störungen wie Morbus Parkinson, Morbus Alzheimer, Chorea Huntington, Amyotrophe Lateralsklerose und Olivopontocerebelläre Degeneration. Erfindungsgemäss können die Verbindungen zur Prävention des postischämischen Zelluntergangs, des Zelluntergangs nach Hirntrauma, bei Schlaganfall, Hypoxie, Anoxie und Hypoglykämie und zur Behandlung der Senilen Demenz, Multiinfarkt Demenz sowie Epilepsie und Muskelspastik verwendet werden. Zu den psychiatrischen Erkrankungen gehören Angstzustände, Schizophrenie, Migräne, Schmerzzustände, sowie die Behandlung von Schlafstörungen und der Entzugssymptomatik nach Drogenmißbrauch wie bei Alkohol-, Kokain-, Benzodiazepin- oder Opiat-Entzug.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelantine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie drüber hinaus Hilfsstoffe wie Konservierungs-, Stabilisierungs-, Netzmittel oder Emulgatoren, Salze zur Veränderung des osmotischen Drucks oder Puffer.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5-1000 mg, vorzugsweise 50-200 mg, wobei die Dosis als einmal zu verabreichende Einzeldosis oder unterteilt in 2 oder mehreren Tagesdosen gegeben werden kann.

Die Herstellung der erfindungsgemäßen Verbindung erfolgt nach an sich bekannten Methoden. Beispielsweise gelangt man zu Verbindungen der Formel I dadurch, daß man
a) eine Verbindung der Formel II 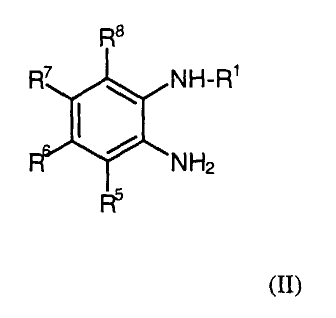 worin R¹ bis R⁸ die obige Bedeutung haben, mit Oxalsäure oder reaktiven Oxalsäurederivaten cyclisiert oder
b) eine Verbindung der Formel III 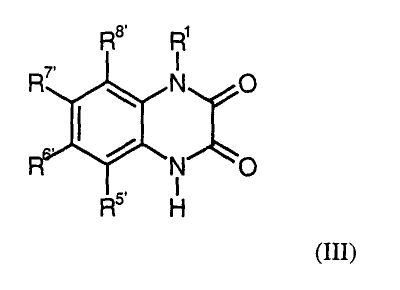 worin R¹ die obige Bedeutung hat und einer der Substituenten R^{5'}, R^{6',} R^{7'}oder R^{8'} eine Fluchtgruppe darstellt, nucleophil substituiert und gewünschtenfalls anschließend die Estergruppe verseift oder die Säuregruppe verestert oder amidiert oder die Nitrogruppe reduziert zur Aminogruppe oder die Aminogruppe alkyliert oder acyliert oder die Aminogruppe gegen Halogen oder Cyano austauscht oder eine Nitrogruppe oder Halogen einführt oder eine Etherspaltung vornimmt oder den Alkohol in Halogenid überführt oder dieses Halogen nukleophil substituiert oder ein Nitril in das Tetrazol überführt oder die Isomeren trennt oder die Salze bildet.

Die Cyclisierung zu Verbindungen der Formel I erfolgt mit Oxalsäure in bekannter Weise einstufig in saurem Milieu oder mit einem reaktiven Oxalsäurederivat einstufig oder auch zweistufig. Als bevorzugt ist das Zweistufenverfahren zu betrachten, bei dem das Diamin mit einem Oxalsäurederivat wie dem Oxalesterhalbchlorid oder reaktiven Oxalsäureimidazolidderivaten in polaren Lösungsmitteln wie cyclischen oder acyclischen Ethern oder halogenierten Kohlenwasserstoffen beispielsweise Tetrahydrofuran, Diethylether oder Methylenchlorid in Gegenwart einer Base wie organischen Aminen beispielsweise Triethylamin, Pyridin, Hünig-Base oder Dimethylaminopyridin umgesetzt wird. Die anschließende Cyclisierung kann basisch oder auch sauer, vorzugsweise aber in saurem Milieu durchgeführt werden, wobei der Reaktionsmischung Alkohol als Lösungsvermittler zugesetzt werden kann.

Geeignete Basen für das Zweistufenverfahren stellen auch Alkalihydride dar wie NaH, die in inerten Lösungsmitteln wie Kohlenwasserstoffen oder Ethern eingesetzt werden.

Als Fluchtgruppen in der Verfahrensvariante b) sowie bei der Herstellung der Ausgangsverbindungen der Formel II sind Halogene wie Fluor, Chlor, Brom, Jod oder O-Mesylat, O-Tosylat, O-Triflat oder O-Nonaflat geeignet. Die nucleophile Substitution wird nach literaturbekanntenMethoden in Gegenwart einer Base durchgeführt und wird durch eine aktivierende elektronenziehende Gruppe wie z.B. Nitro, Cyano, Trifluormethyl vorzugsweise in o-Stellung begünstigt.

Als Nucleophile sind beispielsweise primäre und sekundäre Amine, N-enthaltende ungesättigte oder gesättigte Heterocyclen, Cyanid, Alkoholate, Thiolate u.a. geeignet. Die Umsetzung kann in polaren Lösungsmitteln wie Alkoholen, halogenierten Kohlenwasserstoffen, Dimethylacetamid, Acetonitril oder Wasser oder ohne Lösungsmittel vorgenommen werden. Als Basen sind anorganische Basen wie Alkali- oder Erdalkalihydroxide oder -carbonate oder organische Basen wie cyclische, alicyclische und aromatische Amine, wie DBU, Hünigbase, Pyridin oder Dimethylaminopyridin geeignet. Als Base kann im Fall von Aminen das Nucleophil selbst im Überschuß benutzt werden, wobei man gegebenenfalls ohne weiteres Lösungsmittel arbeiten kann. Bei zu niedrigem Siedepunkt des Amins kann gegebenenfalls unter Druck im Autoklaven gearbeitet werden.

Die sich gegebenenfalls anschließende Verseifung einer Estergruppe kann basisch oder vorzugsweise sauer erfolgen, indem man bei erhöhter Temperatur bis zur Siedetemperatur des Reaktionsgemisches in Gegenwart von Säuren wie hoch konzentrierter wäßriger Salzsäure gegebenenfalls in Lösungsmitteln wie beispielsweise Trifluoressigsäure oder Alkoholen hydrolysiert. Phosphonsäureester werden bevorzugt durch Erhitzen in hochkonzentrierten wäßrigen Säuren wie zum Beispiel konzentrierter Salzsäure gegebenenfalls unter Zusatz eines Alkohols oder durch Behandlung mit Trimethylsilylhalogenid in inerten Lösungsmitteln wie z.B. Acetonitril und anschließende Behandlung mit Wasser hydrolysiert.

Die Veresterung der Carbonsäure oder Phosphonsäure geschieht in an sich bekannter Weise mit dem entsprechenden Alkohol unter Säurekatalyse oder in Gegenwart eines aktivierten Säurederivats. Als aktivierte Säurederivate kommen zum Beispiel Säurechlorid, -imidazolid oder -anhydrid in Frage. Bei den Phosphonsäuren kann man die Veresterung durch Umsetzung mit Orthoestern gegebenenfalls unter Zusatz von Katalysatoren wie p-Toluolsulfonsäure erreichen.

Die Amidierung erfolgt an den freien Säuren oder an deren reaktiven Derivaten wie beispielsweise Säurechloriden, gemischten Anhydriden, Imidazoliden oder Aziden durch Umsetzung mit den entsprechenden Aminen bei Raumtemperatur.

Die Reduktion der Nitrogruppe zur Aminogruppe erfolgt katalytisch in polaren Lösungsmitteln bei Raumtemperatur oder erhöhter Temperatur. Als Katalysatoren sind Metalle wie Raney-Nickel oder Edelmetallkatalysatoren wie Palladium oder Platin gegebenenfalls auf Trägern geeignet. Statt Wasserstoff kann auch Ammoniumformiat in bekannter Weise benutzt werden. Reduktionsmittel wie Zinn-II-chlorid oder Titan-III-chlorid können ebenso verwendet werden wie komplexe Metallhydride eventuell in Gegenwart von Schwermetallsalzen. Als Reduktionsmittel ist auch Eisen nutzbar. Die Reaktion wird dann in Gegenwart einer Säure wie z.B. Essigsäure oder Ammoniumchlorid gegebenenfallls unter Zusatz eines Lösungsmittels wie Wasser durchgeführt. Es kann vorteilhaft sein vor der Reduktion die Estergruppe einzuführen. Bei Vorhandensein mehrerer Nitrogruppen im Molekül kann die gewünschte orthoständige Nitrogruppe auch selektiv in üblicher Weise mit Na₂S reduziert werden.

Wird eine Alkylierung einer Aminogruppe gewünscht, so kann nach üblichen Methoden beispielsweise mit Alkylhalogeniden oder nach der Mitsonubo Variante durch Umsetzung mit einem Alkohol in Gegenwart von Triphenylphosphin und Azodicarbonsäureester alkyliert werden oder man unterwirft das Amin einer reduktiven Aminierung mit Aldehyden oder Ketonen gegebenenfalls nacheinander mit zwei verschiedenen Carbonylverbindungen, wobei man gemischte Derivate erhält (Literatur z.B. Verardo et al. Synthesis 1993, 121; Synthesis 1991, 447; Kawaguchi, Synthesis 1985, 701; Micovic et al. Synthesis 1991, 1043).

Die Acylierung einer Aminogruppe erfolgt in üblicher Weise beispielsweise mit einem Säurehalogenid oder Säureanhydrid gegebenenfalls in Gegenwart einer Base wie Dimethylaminopyridin in Lösungsmitteln wie Methylenchlorid, Tetrahydrofuran oder Pyridin oder nach der Scotten Baumann Variante in wässriger Lösung bei schwach alkalischem pH-Wert.

Die Einführung der Cyanogruppe kann mit Hilfe der Sandmeyer-Reaktion erfolgen; beispielsweise kann man die aus den Aminoverbindungen mit Nitriten intermediär gebildeten Diazoniumsalze mit Alkalicyaniden in Gegenwart von Cu-I-cyanid umsetzen.

Die Einführung der Halogene Chlor, Brom oder Jod über die Aminogruppe kann beispielsweise auch nach Sandmeyer erfolgen, indem man die mit Nitriten intermediär gebildete Diazoniumsalze mit Cu(I)chlorid oder Cu(I)bromid in Gegenwart der entsprechenden Säure wie Salzsäure oder Bromwasserstoffsäure oder mit Kaliumjodid umsetzt.
Wenn ein organischer Salpetrigsäureester benutzt wird, kann man die Halogene z.B. durch Zusatz von Methylenjodid oder Tetrabrommethan einführen in einem Lösungsmittel wie zum Beispiel Dimethylformamid.
Die Einführung von Fluor gelingt beispielsweise durch Balz Schiemann-Reaktion des Diazoniumtetrafluorborates.

Die Einführung einer NO₂-Gruppe gelingt durch eine Reihe von bekannten Nitrierungsmethoden. Beispielsweise kann mit Nitroniumtetrafluoroborat in inerten Lösungsmitteln wie halogenierten Kohlenwasserstoffen oder in Sulfolan, Eisessig oder Acetonitril nitriert werden. Möglich ist auch die Einführung z.B. durch Nitriersäure in konz. Schwefelsäure als Lösungsmittel bei Temperaturen zwischen 0°C und 30°C.
Die Einführung von Halogen gelingt durch bekannte Halogenierungsmethoden wie z.B. durch elektrophile aromatische Substitution.
Beispielsweise kann nach einem Verfahren mit Jod und Jodsäure von Wirth et al. [Liebigs Ann. Chem. 634, 84 (1960)] oder mit N-Jodsuccinimid in Lösungsmitteln wie Tetrahydrofuran, Dimethylformamid oder Trifluormethansulfonsäure jodiert werden.

Die Etherspaltung erfolgt nach den üblichen Methoden beispielsweise durch Reaktion mit Trimethylbromsilan gegebenenfalls unter Zusatz von Alkaliiodid in einem inerten Lösungsmittel wie Acetonitril bei einer Temperatur von 0°C bis zur Siedetemperatur des Lösungsmittels.

Die Einführung des Tetrazoles gelingt durch Umsetzung des entsprechenden Nitriles mit einem Azid wie z.B. Trimethylsilylazid, Stickstoffwasserstoffsäure oder Natriumazid gegebenenfalls unter Zusatz einer Protonenquelle wie z.B. Ammoniumchlorid oder Triethylammoniumchlorid in polaren Lösungsmitteln wie Dimethylformamid, Dimethylacetamid oder N-Methylpyrrolidon bei Temperaturen bis zum Siedepunkt des Lösungsmittels.

Die Überführung des Alkohols in das Halogenid gelingt mit Säurehalogeniden wie Thionylchlorid oder Phosphortribromid mit oder ohne Lösungsmittel. Als Lösungsmittel sind halogenierte Kohlenwasserstoffe, z.B. Methylenchlorid oder Ether möglich.

Die Isomerengemische können nach üblichen Methoden wie beispielsweise Kristallisation, Chromatographie oder Salzbildung in die Enantiomeren bzw. E/Z-Isomeren aufgetrennt werden.

Die Herstellung der Salze erfolgt in üblicher Weise, indem man eine Lösung der Verbindung der Formel I mit der äquivalenten Menge oder einem Überschuß einer Base oder Säure, die gegebenenfalls in Lösung ist, versetzt und den Niederschlag abtrennt oder in üblicher Weise die Lösung aufarbeitet.

Soweit die Herstellung der Ausgangsverbindungen nicht beschrieben wird, sind diese bekannt oder analog zu bekannten Verbindungen, beispielsweise nach WO93/08171 oder hier beschriebenen Verfahren herstellbar.

Die nachfolgenden Beispiele sollen das erfindungsgemäße Verfahren erläutern:

### Herstellung der Ausgangsverbindungen

### 1. A)

2,05g Mono-tert.butoxycarbonylethylendiamin werden mit 1,25g (0,86ml) 4-Fluor-3-nitro-1-trifluormethylbenzol 30 min. auf 50°C Badtemperatur erwärmt. Die Masse verfestigt sich dabei. Anschließend wird über Kieselgel mit Methylenchlorid:Ethanol=10:1 chromatographiert. Man erhält 2,2g [1-N-Carbo t-butoxy 2 N-(2-nitro-4-trifluormethylphenyl)]ethylendiamin.

### B)

N-(2-Nitro-4-trifluormethyl)ethylendiamin, 2,2 g 1-N-Carbo-t-butoxy-2-N-(2-nitro-4-trifluormethylphenyl)ethylendiamin werden in 60ml Ethanol mit 60ml 1-N-Salzsäure 2h auf 110°C erwärmt. Nach Einengen erhält man 1,77g N-(2-Nitro-4-trifluormethylphenyl)ethylendiamin als Hydrochlorid.

### C)

[N-1-(Benzoyl)-N-2-(2-Nitro-4-trifluormethylphenyl)]ethylendiamin. 570mg N-(2-Nitro-4-trifluormethylphenyl)ethylendiamin hydrochlorid werden in 15ml Methylenchlorid zunächst mit 424mg Triethylamin und dann mit 295mg Benzoylchlorid versetzt. Nach 2h Rühren bei Raumtemperatur wird zweimal mit Wasser extrahiert. Die organische Phase wird getrocknet, filtriert und eingeengt. Man erhält 690mg [N-1-(Benzoyl)-N-2-(2-Nitro-4-trifluormethylphenyl)] ethylendiamin.

Analog wird hergestellt
[N-1-(Methansulfonyl)-N-2-(-2-Nitro-4-trifluormethylphenyl)]ethylendiamin.
N-1-(4-Chlor-benzoylamino)-N-2-(2-nitro-4-trifluormethylphenyl) ethylendiamin.
N-1-Nicotinoylamino)-N-2-(2-nitro-4-trifluormethylphenyl) ethylendiamin
N-1-(Phenylsulfonylamino)-N-2-(2-nitro-4-trifluormethylphenyl) ethylendiamin

### 2. A) 1-(2-Nitro-4-trifluormethylphenylamino)-2-methoxyethan

3,75g 2-Methoxyethylamin und 10,5g 4-Fluor-3-nitro-1-trifluormethylbenzol werden in 200ml Wasser mit 10g Natriumcarbonat 2h auf 100°C Badtemperatur erwärmt. Beim Abkühlen fällt das Produkt aus, das sich durch Absaugen isolieren läßt (9,8g). Die wässrige Mutterlauge wird mit 4-N-Salzsäure ausgesäuert und dreimal mit je 100ml Essigester extrahiert. Die gesammelten organischen Extrakte werden getrocknet, filtriert und eingeengt. Man erhält nochmals 2,7g. Gesamtausbeute 12,5g 1-(2-Nitro-4-trifluormethylphenyl)amino-2-methoxyethan.

### 3. A)

1,1g 2,4-Difluornitrobenzol werden mit 2,8g Aminomethanphosphonsäurediethylester 2h auf 40°C erwärmt. Anschließend wird über Kieselgel mit Methylenchlorid:Ethanol=10:1 chromatographiert. Man erhält 1,6g N-(2-Nitro-5-fluorphenyl)aminomethanphosphonsäurediethylester.

Analog wird hergestellt.
N-(2-Nitro-4-fluorphenyl)-aminomethanphosphonsäurediethylester
1-[N-(2-Nitro-4-fluorphenyl)amino]-ethanphosphonsäurediethylester
1-[N-(2-Nitro-5-fluorphenyl)-amino]-ethanphosphonsäurediethylester
1-(2-Nitro-4-trifluormethylphenylamino)-4-methoxypropanphosphonsäurediethylester.

### B)

331mg N-Benzophenoniminylmethanphosphonsäurediethylester werden mit 40mg Aliquat 336 und mit 209mg 2-Methoxy-1-bromethan vorgelegt und bei 0°C mit 280mg gepulvertem Kaliumhydroxyd versetzt; anschließend wird bei Raumtemperatur 3,5h gerührt. Der Ansatz wird mit Methylenchlorid und 180mg Kieselgel versetzt, kurz gerührt und abgesaugt. Das eingeengte Filtrat wird über Kieselgel mit Cyclohexan:Essigester=1:1 chromatographiert. Man erhält 180mg 2-(N-Benzophenoniminyl)-4-methoxypropanphosphonsäurediethylester.

### C)

2,0g 2-(N-Benzophenoniminyl)-4-methoxy-propanphosphonsäurediethylester werden in 30ml 1-N-HCl und 30ml Diethylether 3h bei Raumtemperatur gerührt. Es wird die organische Phase abgetrennt und die Wasserphase nochmals mit Diethylether extrahiert. Die organische Phase enthält Benzophenon und wird verworfen. Die wässrige Phase wird zur Trockene eingeengt, in 15ml gesättigter Kochsalzlösung aufgenommen, mit Na₂CO₃ neutralisiert und dreimal mit 50ml Methylenchlorid extrahiert. Die organische Phase wird getrocknet, filtriert und eingeengt und ergibt 800mg 2-Amino-4-methoxypropanphosphonsäurediethylester.

### 4. A)

790mg N-(2-Nitro-5-fluorphenyl) aminomethanphosphonsäurediethylester werden in 50ml Ethanol mit 2,5g Raney Nickel versetzt und unter Wasserstoffnormaldruck bei Raumtemperatur 2h hydriert. Nach Absaugen vom Katalysator wird eingeengt. Man erhält 660mg N-(2-Amino-5-fluorphenyl) aminomethanphosphonsäurediethylester.
Analog werden hergestellt
N-(2-Amino-4-fluorphenyl) aminomethanphosphonsäurediethylester,
1-[(2-Amino-4-trifluormethylphenyl) amino*]-4-*methoxypropanphosphonsäurediethylester
1-[N-(2-Amino-4-fluorphenyl)amino]-ethanphosphonsäurediethylester
1-[N-(2-Amino-5-fluorphenyl)-amino]-ethanphosphonsäurediethylester
1-(2-Amino-4-trifluormethylphenyl) amino-2-methoxyethan
N-1-(Methansulfonyl)-N-2-(-2-amino-4-trifluormethylphenyl) ethylendiamin
N-1-(benzoylamino)-N-2-(2-amino-4-trifluormethylphenyl) ethylendiamin
N-1-(4-Chlor-benzoylamino)-N-2-(2-amino-4-trifluormethylphenyl) ethylendiamin.
N-1-Nicotinoylamino)-N-2-(2-amino-4-trifluormethylphenyl) ethylendiamin
N-1-(Phenylsulfonylamino)-N-2-(2-amino-4-trifluormethylphenyl) ethylendiamin

### Beispiel 1

### (7-Fluor-1,2,3,4-tetrahydro-2,3-dioxochinoxalin-1-yl)-methanphosphonsäurediethylester

660mg N(-2 Amino-5-fluorphenyl)-aminomethanphosphonsäurediethylester werden in 70ml absoluten Tetrahydrofuran mit 509mg Triethylamin vorgelegt. Zu dieser Lösung wird langsam eine Lösung von 685mg Oxalsäureethylesterchlorid in 30ml Tetrahydrofuran getropft. Der Ansatz wird 4h bei Raumtemperatur gerührt. Nach Absaugen der ausgefallenen Salze wird das Filtrat eingeengt, in einem Gemisch von 23ml Ethanol und 23ml 1-N-Salzsäure für 2h bei 110°C Badtemperatur gekocht. Es wird zur Trockene eingeengt und der Rückstand über Kieselgel mit Methylenchlorid:Ethanol=10:1 chromatographiert. Man erhält 561mg (7-Fluor-1,2,3,4-tetrahydro-2,3-dioxochinoxalin-1-yl) methanphosphonsäurediethylester.

Analog werden hergestellt:
(6-Fluor-1,2,3,4-tetrahydro-2,3-dioxo chinoxalin-1-yl)methanphosphonsäurediethylester
1-[(7-Fluor-1,2,3,4-tetrahydro-2,3-dioxo chinoxalin-1-yl)]-ethanphosphonsäurediethylester
1-[(6-Fluor-1,2,3,4-tetrahydro-2,3-dioxo chinoxalin-1-yl)] ethanphosphonsäurediethylester

Grundsätzlich analog werden hergestellt:
6-Trifluormethyl-1-(1-methoxyeth-2-yl)-1,2,3,4-tetrahydro-2,3-dioxo-chinoxalin.
6-Trifluormethyl-1-(1-N-benzoylaminoeth-2-yl)-1,2,3,4-tetrahydro-2,3-dioxo-chinoxalin
6-Trifluormethyl-1-(1-N-methansulfonylaminoeth-2-yl)-1,2,3,4-tetrahydro-2,3-dioxo-chinoxalin
1-(6-Trifluormethyl-1,2,3,4-tetrahydro-2,3-dioxo-chinoxalin-1-yl)-3-methoxy propanphosphonsäurediethylester.
6-Trifluormethyl-1-(1-N-phenylsulfonylaminoeth-2-yl)-1,2,3,4-tetrahydro-2,3-dioxo-chinoxalin
6-Trifluormethyl-1-(1-N-nicotinoylamino-2-yl)-1,2,3,4-tetrahydro-2,3-dioxo-chinoxalin
6-Trifluormethyl-1-(1-N-4-chlor-benzoylaminoeth-2-yl)-1,2,3,4-tetrahydro-2,3-dioxo-chinoxalin

### Beispiel 2

1g 6-Trifluormethyl-1-(1-methoxyeth-2-yl)-1,2,3,4-tertrahydro-2,3-dioxo-chinoxalin werden in 20ml absoluten Acetonitril mit 3,15ml Trimethylbromsilan und 1,4g Natriumjodid versetzt und 1h auf 80°C Badtemperatur erwärmt. Nach Zugabe von 25ml Wasser wird mit Essigester extrahiert. Die organische Phase wird abgetrennt, eingeengt und über Kieselgel mit Toluol:Eisessig:Wasser=10:10:1 chromatographiert. Nach Einengen der entsprechenden Fraktionen und Ausrühren mit Ethanol erhält man 330mg 6-Trifluormethyl-1-(1-hydroxyeth-2-yl)-1,2,3,4-tetrahydro-2,3-dioxo-chinoxalindion.

### Beispiel 3

615mg (7-Fluor-1,2,3,4-tetrahydro-2,3-dioxo-chinoxalin-1yl-) methanphosphonsäurediethylester werden in 60ml Methylenchlorid mit 743mg Nitroniumtetrafluorborat versetzt. Der Ansatz wird 2h bei Raumtemperatur gerührt. Es wird mit 50ml Wasser versetzt und nach Abtrennen der organischen Phase dreimal mit Methylenchlorid extrahiert. Die gesammelte organische Phase wird getrocknet, filtriert und eingeengt. Der Rückstand wird über Kieselgel mit Methylenchlorid:Ethanol=10:1 chromatographiert. Man erhält 350mg (6-Nitro-7-Fluor-1,2,3,4-tetrahydro-2,3-dioxochinoxalin-1-yl)-methanphosphonsäurediethylester.

Grundsätzlich analog werden hergestellt:
(6-Fluor-7-nitro-1,2,3,4-tetrahydro-2,3-dioxochinoxalin-1-yl) methanphosphonsäurediethylester.
1-[(6-Fluor-7-Nitro-1,2,3,4-tetrahydro-2,3-dioxochinoxalin-1-yl)] ethanphosphonsäurediethylester.
1-[(7-Fluor-6-Nitro-1,2,3,4-tetrahydro-2,3-dioxochinoxalin-1-yl)] ethanphosphonsäurediethylester.
1-[(6-Trifluormethyl-7-Nitro-1,2,3,4-tetrahydro-2,3-dioxochinoxalin-1-yl)] ethanphosphonsäurediethylester
N-[(6-Trifluormethyl-7-Nitro-1,2,3,4-tetrahydro-2,3-dioxochinoxalin-1-yl)] methanphosphonsäurediethylester
N-[(6-Trifluormethyl-7-Nitro-1,2,3,4-tetrahydro-2,3-dioxochinoxalin-1-yl)] acetonitril

### Beispiel 4

140mg (6-Nitro-7-fluor-1,2,3,4-tetrahydro-2,3-dioxochinoxalin-1-yl) methanphosphonsäurediethylester werden mit 129mg Morpholin 1,5h auf 120°C Badtemperatur erwärmt. Nach Einengen im Vakuum wird der Rückstand über Kieselgel mit Toluol:Eisessig:Wasser = 10:10:1 chromatographiert. Nach Einengen der entsprechenden Fraktionen erhält man 300mg (7-Morpholino-6-nitro-1,2,3,4-tetrahydro-2,3-dioxochinoxalin-1-yl) methanphosphonsäurediethylester.

Analog wird hergestellt:
[6-(N-Imidazolyl)-7-nitro-1,2,3,4-tetrahydro-2,3-dioxochinoxalin-1-yl] methanphosphonsäurediethylester.
1-[6-(N-Imidazolyl)-7-nitro-1,2,3,4-tetrahydro-2,3-dioxochinoxalin-1-yl] ethanphosphonsäurediethylester
1-[7-(N-Imidazolyl)-6-nitro-1,2,3,4-tetrahydro-2,3-dioxochinoxalin-1-yl] ethanphosphonsäurediethylester
(6-Morpholino-7-nitro-1,2,3,4-tetrahydro-2,3-dioxochinoxalin-1-yl) methanphosphonsäurediethylester
1-[(6-Morpholino-7-nitro-1,2,3,4-tetrahydro-2,3-dioxochinoxalin-1-yl)] ethanphosphonsäurediethylester
1-[(7-Morpholino-6-nitro-1,2,3,4-tetrahydro-2,3-dioxochinoxalin-1-yl)] ethanphosphonsäurediethylester
1-[(7-2-(Methoxyethylamino-6-nitro-1,2,3,4-tetrahydro-2,3-dioxochinoxalin-1-yl)] methanphosphonsäurediethylester
1-[(7-N-Methylpiperazinyl-6-nitro-1,2,3,4-tetrahydro-2,3-dioxochinoxalin-1-yl)] methanphosphonsäurediethylester
1-[(4-Methylimidazol-1-yl)-7-nitro-1,2,3,4-tetrahydro-2,3-dioxochinoxalin-1-yl] methanphosphonsäurediethylester
1-[(2-Methylimidazol-1-yl)-7-nitro-1,2,3,4-tetrahydro-2,3-dioxochinoxalin-1-yl] methanphosphonsäurediethylester
1-[(2,4-Dimethylimidazol-1-yl)-7-nitro-1,2,3,4-tetrahydro-2,3-dioxochinoxalin-1-yl] methanphosphonsäurediethylester
(7-Thiomorpholino-6-nitro-1,2,3,4-tetrahydro-2,3-dioxochinoxalin-1-yl) methanphosphonsäurediethylester.
(7-NN-Dipropylamino-6-nitro-1,2,3,4-tetrahydro-2,3-dioxochinoxalin-1-yl) methanphosphonsäurediethylester
(7-N,N-Dipropylamino-6-nitro-1,2,3,4-tetrahydro-2,3-dioxochinoxalin-1-yl) ethanphosphonsäurediethylester
(7-N-Methyl-N-propylamino-6-nitro-1,2,3,4-tetrahydro-2,3-dioxochinoxalin-1-yl) methanphosphonsäurediethylester
N-[7-(1,2,4-Triazol-1-yl)-6-nitro-1,2,3,4-tetrahydro-2,3-chinoxalin-1-yl]methanphosphonsäurediethylester

### Beispiel 5

375mg (7-Fluor-6-nitro-1,2,3,4-tetrahydro-2,3-dioxochinoxalin-1-yl)methanphosphonsäurediethylester werden zu einer Lösung gegeben, die aus 200mg Trifluorethanol 60mg Natriumhydrid (80%ig) in 20ml absoluten Tetrahydrofuran hergestellt wurde. Nach der Zugabe wird 4,5h auf 70°C Badtemperatur geheizt. Es wird eingeengt in 50ml Wasser aufgenommen, mit 1N-Salzsäure sauer gestellt und dreimal mit Essigester extrahiert. Die organische Phase wird getrocknet, filtriert und eingeengt. Der Rückstand wird über Kieselgel mit Toluol:Eisessig:Wasser=10:10:1 chromatographiert. Nach Einengen der entsprechenden Fraktionen und Ausrühren mit Ethanol erhält man in Form des Rückstandes 19mg (6-Nitro-7-trifluorethoxy-1,2,3,4-tetrahydro-2,3-dioxo-chinoxalin-1-yl)-methanphosphonsäurediethylester.

### Beispiel 6

259mg (6-Nitro-7-morpholino-1,2,3,4-tetrahydro-2,3-dioxo-chinoxalin-1-yl)-methanphosphonsäurediethylester werden in 10ml absoluten Acetonitril mit 628mg Trimethylbromsilan versetzt und 1h bei Raumtemperatur gerührt. Es wird wenig Wasser zugegeben und zur Trockene eingeengt. Der Rückstand wird über silanisiertes Kieselgel mit Methanol als Elutionsmittel chromatographiert. Man erhält 60mg (6-Nitro-7-morpholino-1,2,3,4-tetrahydro-2,3-dioxo-chinoxalin-1-yl)methanphosphonsäure.

Analog werden hergestellt:
1-(6-Trifluormethyl-1,2,3,4-tetrahydro-2,3-dioxochinoxalin-1-yl)-3-methoxypropanphosphonsäure.
(6-Morpholino-7-nitro-1,2,3,4-tetrahydro-2,3-dioxochinoxalin-1-yl) methanphosphonsäure
1-[(6-Morpholino-7-nitro-1,2,3,4-tetrahydro-2,3-dioxochinoxalin-1-yl)] ethanphosphonsäure
1-[(7-Morpholino-6-nitro-1,2,3,4-tetrahydro-2,3-dioxochinoxalin-1-yl)] ethanphosphonsäure
1-[(7-(2-Methoxyethylamino-6-nitro-1,2,3,4-tetrahydro-2,3-dioxochinoxalin-1-yl)] methanphosphonsäure
1-[(7-N-Methylpiperazinyl-6-nitro-1,2,3,4-tetrahydro-2,3-dioxochinoxalin-1-yl)] methanphosphonsäure
(7-Thiomorpholino-6-nitro-1,2,3,4-tetrahydro-2,3-dioxochinoxalin-1-yl) methanphosphonsäure.
[(6-Trifluormethyl-7-morpholino-chinoxalin-2,3-dion)-1-yl]-methanphosphonsäure;
   Schmelzpunkt > 300°C, ¹H-NMR in DMSO: 7,9 s, 1H; 7,4 s, 1H; 4,6 d, 11Hz, 2H: 3,7 t 5Hz, 2H; 2,9 t, 5Hz, 2H.

In analoger Weise werden hergestellt:
[(6-Trifluormethyl-7-[piperidin-1-yl]chinoxalin-2,3-dion)-1-yl]-methanphosphonsäure;
   Schmelzpkt > 300°C;
[(6-Trifluormethyl-7-[2,6-dimethyl-(morpholin-1-yl)]chinoxalin-2,3-dion)-1yl]-methanphosphonsäure;
[(6-Trifluormethyl-7-(hexahydroazepin-1-yl)chinoxalin-2,3-dion)-1-yl]-methanphosphonsäure;
[(6-Trifluormethyl-7-[(4-phenylpiperazin-1-yl)chinoxalin-2,3-dion)-1-yl]-methanphosphonsäure;
1-[(6-Trifluormethyl-7-[morpholin-1-yl]chinoxalin-2,3-dion)-1-yl]-ethanphosphonsäure;

### Beispiel 7

250mg [(6-(N-Imidazolyl)-7-nitro-1,2,3,4-tetrahydro-2,3-dioxochinoxalin-1-yl] methanphosphonsäurediethylester werden in 3ml konzentrierter Salzsäure 2,5h auf 110°C Badtemperatur erwärmt. Nach Einengen wird in Wasser aufgenommen und das ausgefallenen Produkt abgesaugt. Man erhält 100mg [6-(N-Imidazolyl)-7-nitro-1,2,3,4-tetrahydro-2,3-dioxochinoxalin-1-yl] methanphosphonsäure

Analog werden hergestellt:
(6-Nitro-7-fluor-1,2,3,4-tetrahydro-2,3-dioxo-chinoxalin-1-yl) methanphosphonsäure
(7-Nitro-6-fluor-1,2,3,4-tetrahydro-2,3-dioxochinoxalin-1-yl) methanphosphonsäure.
1-[6-(N-Imidazolyl)-7-nitro-1,2,3,4-tetrahydro-2,3-dioxochinoxalin-1-yl] ethanphosphonsäure
1-[7-(N-Imidazolyl)-6-nitro-1,2,3,4-tetrahydro-2,3-dioxochinoxalin-1-yl] ethanphosphonsäure
1-[(6-Fluor-7-Nitro-1,2,3,4-tetrahydro-2,3-dioxochinoxalin-1-yl)] ethanphosphonsäure.
1-[(7-Fluor-6-Nitro-1,2,3,4-tetrahydro-2,3-dioxochinoxalin-1-yl)] ethanphosphonsäure.
1-[7-(2-Methylimidazol-1-yl)-6-nitro-1,2,3,4-tetrahydro-2,3-dioxochinoxalin-1-yl] methanphosphonsäure
1-[7-(4-Methylimidazol-1-yl)-6-nitro-1,2,3,4-tetrahydro-2,3-dioxochinoxalin-1-yl] methanphosphonsäure
1-[7-(2,4-Dimethylimidazol-1-yl)-6-nitro-1,2,3,4-tetrahydro-2,3-dioxochinoxalin-1-yl] methanphosphonsäure
(7-NN-Dipropylamino-6-nitro-1,2,3,4-tetrahydro-2,3-dioxochinoxalin-1-yl) methanphosphonsäure
(7-N,N-Dipropylamino-6-nitro-1,2,3,4-tetrahydro-2,3-dioxochinoxalin-1-yl) methanphosphonsäure
(7-N-Methyl-N-propylamino-6-nitro-1,2,3,4-tetrahydro-2,3-dioxochinoxalin-1-yl) methanphosphonsäure
1-[(6-Trifluormethyl-7-imidazolyl-chinoxalin-2,3-dion)-1-yl]ethanphosphonsäure.
1-[(6-Trifluormethyl-7-(-4-methylimidazol-1-yl-chinoxalin-2,3-dion)-1yl]methanphosphon-säure
N-[6-Trifluormethyl-7-amino-1,2,3,4-tetrahydro-2,3-dioxo-chinoxalin-1yl]-methanphosphonsäure.
1-[6-Trifluormethyl-7-amino-1,2,3,4-tetrahydro-2,3-dioxo-chinoxalin-1yl]-ethanphosphonsäure.
1-[(6-Trifluormethyl-7-[piperidin-1-yl]chinoxalin-2,3-dion)-1yl]-ethanphosphonsäure
1-[(6-Trifluormethyl-7-[piperidin-1-yl]chinoxalin-2,3-dion)-1yl]-essigsäure
N-[6-Trifluormethyl-7-amino-1,2,3,4-tetrahydro-2,3-dioxo-chinoxalin-1-yl]-methanphosphonsäure
N-[6-Trifluormethyl-7-amino-1,2,3,4-tetrahydro-2,3-dioxochinoxalin-1-yl]-essgsäure
1-[6-Trifluormethyl-7-propylamino-1,2,3,4-tetrahydro-2,3-dioxo-chinoxalin-1-yl]-ethan-1-phosphonsäure
1-[6-Trifluormethyl-7-hexylamino-1,2,3,4-tetrahydro-2,3-dioxo-chinoxalin-1-yl]-ethan-1-phosphonsäure
N-[(6-Trifluormethyl-7-[hex-1-ylamino]-chinoxalin-2,3-dion)-1yl]-methanphosphonsäure
N-[(6-Trifluormethyl-7-[pent-1-ylamino]-chinoxalin-2,3-dion)-1yl]-methanphosphonsäure
N-[(6-Trifluormethyl-7-[hex-2-ylamino]-chinoxalin-2,3-dion)-1yl]-methanphosphonsäure
N-[7-1,2,3,4-Triazol-1-yl-6-nitro-1,2,3,4-tetrahydro-2,3-chinoxalin-1-yl]methanphosphonsäure

### Beispiel 8

### N-(6 Trifluormethyl-7-morpholinochinoxalin-2,3-dion-1-yl)-methanphosphonsäurediethylester

### A

3,3 g (30 mmol) Aminomethanphosphonsäure werden in 120 ml Wasser und 120 ml Ethanol zusammen mit 3,37 g (31,8 mmol) Soda vorgelegt und mit 7,8 g (97%ig, 30 mmol) 3-Trifluormethyl-4,6-dichlornitrobenzol versetzt und 4 h bei 120°C Badtemperatur am Rückfluss gerührt. Nach Abziehen des Ethanol am Rotationsverdampfer wird dreimal mit 100 ml Essigester extrahiert. Die organische Phase wird mit wenig Wasser gewaschen. Sie enthält Ausgangsmaterial und wird verworfen. Die gesammelte wässrige Phase wird mit 4N-Salzsäure sauer auf pH1 gestellt und dreimal mit je 100 ml Essigester extrahiert. Die organische Phase wird mit Wasser gewaschen, getrocknet, filtriert und eingeengt. Man erhält 6,85 g (68% d. Th.) N-(2-Nitro-4-trifluormethyl-5-chlor-phenyl)-aminomethanphosphonsäure vom Schmelzpunkt 207,3°C,.

Analog wird hergestellt:
1-[(2-Nitro-4-trifluormethyl-5-chlor-phenyl)-amino]ethanphosphonsäure

### B,C

6,85g (20,5 mmol) N-(2-Nitro-4-trifluormethyl-5-chlor-phenyl)-aminomethanphosphonsäure werden in 20 ml Morpholin 3,5 h bei 120°C Badtemperatur gerührt. Es wird am Rotationsverdampfer zur Trockene eingeengt und der Rückstand mit 100 ml Orthoameisensäuretriethylester und 779 mg (4.1 mmol) p-Toluolsulfonsäure 3.8 h auf 150°C Badtemperatur erwärmt. Nach Einengen am Rotatiosverdampfer zur Trockene wird in 100 ml Wasser aufgenommen, mit Kochsalz versetzt und dreimal mit je 100 ml Essigester extrahiert. Die gesammelte Essigesterphase wird mit verdünnter Kochsalzlösung gewaschen, getrocknet, filtriert und eingeengt. Ausbeute 10,6g (> 100% d.Th., enthält noch Morpholinhydrochlorid) an N-(2-Nitro-4-trifluormethyl-5-morpholino-phenyl)-amino methanphosphonsäurediethylester.

### D

10,6g (∼20 mmol) N-(2-Nitro-4-trifluormethyl-5-morpholino-phenyl)-aminomethanphosphonsäurediethylester werden in 250 ml Etanol mit 4,5g Palladium auf Kohle (10%ig) unter Wasserstoffnormaldruck bei Raumtemperatur 3,5h hydriert. Nach Absaugen des Katalysators über Kieselgur und Einengen des Filtrates erhält man 9,2g (>100% d.Th., enthält noch Morpholinhydrochlorid) an N-(2-Amino-4-trifluormethyl-5-morpholinophenyl)-aminomethanphosphonsäurediethylester.

In analoger Weise über B, C, D werden hergestellt:
N-(2-Amino-4-trifluormethyl-5-piperidino-phenyl)-aminomethanphosphonsäurediethylester
N-(2-Amino-4-trifluormethyl-5-(-2,6-dimethylmorpholino)-phenyl)-aminomethanphosphonsäurediethylester
N-(2-Amino-4-trifluormethyl-5-hexahydroazepino-phenyl)-aminomethanphosphonsäurediethylester
N-(2-Amino-4-trifluormethyl-5-phenylpiperazinyl-phenyl)-aminomethanphosphonsäurediethylester
1-[(2-Amino-4-trifluormethyl-5-morpholinophenyl)-amino]ethanphosphonsäurediethylester
N[(2-Amino-4-trifluormethyl-5-hex-1-ylaminophenyl)-amino]methanphosphorsäurediethyl-ester
N[(2-Amino-4-trifluormethyl-5-pent-1-ylaminophenyl)-amino]methanphosphorsäurediethyl-ester
N[(2-Amino-4-trifluormethyl-5-hex-2-ylaminophenyl)-amino]methanphosphorsäurediethyl-ester

### E

1,26g (2,7 mmol) N-(2-Amino-4-trifluormethyl-5-morpholino-phenyl)-aminomethanphosphonsäurediethylester werden in 60 ml absolutem Tetrahydrofuran zusammen mit 0,79 ml (5,7 mmol) Triethylamin vorgelegt. Die Mischung wird tropfenweise mit einer Lösung von 0,63ml (5,7 mmol) Oxalsäureetylesterchlorid in 20ml Tetrahydrofuran versetzt. Anschliessend wird 3 h bei Raumtemperatur gerührt. Der Ansatz wird abgesaugt und das Filtrat eingeengt. Der Rückstand des eingeengten Filtrates wird in 20 ml Ethanol und 20ml 1-N Salzsäure aufgenommen und 2h bei 110°C Badtemperatur gerührt. Nach Abziehen des Ethanols am Vakuum wird mit Wasser auf 30 ml verdünnt und dreimal mit je 30 ml Essigester ausgeschüttelt. Die gesammelte Essigesterphase wird einmal mit 30 ml Wasser gewaschen, getrocknet, filtriert und eingeengt. Man erhält 1,13 g (89,7% d.Th.) [(6 Trifluormethyl-7-morpholinochinoxalin-2,3-dion)-1yl]methanphosphonsäurediethylester vom Schmelzpunkt 192,6°C.

In analoger Weise werden hergestellt:
[(6-Trifluormerhyl-7-[piperidin-1-yl]chinoxalin-2,3-dion)-1yl]-methanphosphonsäurediethylester;
[(6-Trifluormethyl-7-[2,6-dimethyl-(morpholin-1-yl)]chinoxalin-2,3-dion)-1yl]-methanphosphonsäurediethylester;
[(6-Trifluormethyl-7-(hexahydroazepin-1-yl)chinoxalin-2,3-dion)-1yl]-methanphosphonsäure-diethylester;
[(6-Trifluormethyl-7-[(4-phenylpiperazin-1-yl)chinoxalin-2,3-dion)-1yl]-methanphosphonsäurediethylester;
1-[(6-Trifluormethyl-7-[morpholin-1-yl]-chinoxalin-2,3-dion)-1yl]-ethanphosphonsäurediethylester;
N-[(6-Trifluormethyl-7-[hex-1-ylamino]-chinoxalin-2,3-dion)-1yl]-methanphosphonsäurediethylester
N-[(6-Trifluormethyl-7-[pent-1-ylamino]-chinoxalin-2,3-dion)-1yl]-methanphosphonsäure-diethylester
N-[(6-Trifluormethyl-7-[hex-2-ylamino]-chinoxalin-2,3-dion)-1yl]-methanphosphonsäurediethylester

### Beispiel 9

### 1-(6 Trifluormethyl-7-imidazolylchinoxalin-2,3-dion-1-yl)-ethanphosphonsäurediethylester

### A

1,67g (5 mmol) 1-[(2-Nitro-4-trifluormethyl-5-chlor-phenyl)amino]ethanphosphonsäure (nach Beispiel 8, **A**) werden zusammen mit 1,70 g Imidazol 4 h bei 160°C Badtemperatur gerührt. Es wird in 100 ml Wasser aufgenommen und zweimal mit Ionenaustauscher IR 120 (stark saure Form, Amberlite, 20-50 mesh) gerührt und abgesaugt. Anschliessend wird am Rotationsverdampfer zur Trockene eingeengt und der Rückstand mit 24 ml Orthoameisensäuretriethylester und 156 mg (0,82 mmol) p-Toluolsulfonsäure 10 h auf 150°C Badtemperatur erwärmt. Nach Einengen am Rotatiosverdampfer zur Trockene wird über Kieselgel mit Methylenchlorid: Ethanol= 10: 1 chromatographiert. Man erhält 222 mg (17%d.Th.) an 1-[(2-Nitro-4-trifluormethyl-5-imidazolylphenyl)-amino]ethanphosphonsäurediethylester.

In analoger Weise werden hergestellt:
1-[(2-Nitro-4-trifluormethyl-5(4-methylimidazol-1-yl)-phenyl)-amino]methanphosphonsäurediethylester

### B

572 mg (1,4 mmol) 1-[(2-Nitro-4-trifluormethyl-5-imidazolyl-phenyl)-amino]ethanphosphonsäurediethylester werden in 60 ml Etanol mit 300 mg Palladium auf Kohle (10%ig) unter Wasserstoffnormaldruck bei Raumtemperatur 1,5h hydriert. Nach Absaugen des Katalysators über Kieselgur und Einengen des Filtrates erhält man 531 mg (99,6% d.Th.) an 1-[(2-Amino-4-trifluormethyl-5-imidazolylphenyl)-amino]ethanphosphonsäureethyl-ester.

In analoger Weise werden hergestellt:
1-[(2-Amino-4-trifluormethyl-5(4-methylimidazol-1-yl)-phenyl)-amino]methanphosphonsäurediethylester

### C

531 mg (1,4 mmol) 1-[(2-Amino-4-trifluormethyl-5-imidazolyl-phenyl)-amino]ethanphosphonsäurediethylester werden in 35 ml absolutem Tetrahydrofuran zusammen mit 0,4 ml (2,9 mmol) Triethylamin vorgelegt. Die Mischung wird tropfenweise mit einer Lösung von 0,32ml (2,9 mmol) Oxalsäureetylesterchlorid in 10 ml Tetrahydrofuran versetzt. Anschliessend wird 3 h bei Raumtemperatur gerührt. Der Ansatz wird abgesaugt und das Filtrat eingeengt. Der Rückstand des eingeengten Filtrates wird in 15 ml Ethanol und 15 ml 1-N Salzsäure aufgenommen und 2h bei 110°C Badtemperatur gerührt. Nach Abziehen des Ethanols am Vakuum wird mit Wasser auf 30 ml verdünnt und dreimal mit je 25 ml Essigester ausgeschüttelt. Die gesammelte Essigesterphase wird einmal mit 30 ml Wasser gewaschen, getrocknet, filtriert und eingeengt. Man erhält 1-[(6-Trifluormethyl-7-imidazolylchinoxalin-2,3-dion)-1yl]ethanphosphonsäurediethylester.

In analoger Weise werden hergestellt:
1-[(6-Trifluormethyl-7-(-4-methylimidazol-1-ylchinoxalin-2,3-dion)-1yl]methanphosphonsäurediethylester

### Beispiel 10

### 2-[(6 Trifluormethylchinoxalin-2,3-dion-1-yl)propionitril]

### A

6g (28,7 mmol) 4-Fluor-3-nitrobenzotrifluorid werden in 120ml Wassser mit 4,08g (28,7 mmol) 3-Aminopropionitril (in Form des Fumarates) und 0,36g (60 mmol) Soda versetzt und 3h auf 110°C Badtemperatur erwärmt. Der ausgefallene Feststoff wird abgesaugt. Man erhält 5,1g (68,5% d.Th.) an 2-N-[(2-Nitro-4-trifluormethylphenyl)amino] propionitril.

### B

2g (7,7 mmol) 2-N[(2-Nitro-4-trifluormethylphenyl)amino]propionitril werden in 200ml Ethanol mit 500mg Palladium auf Aktivkohle (10%) 1,5h bei Raumtemperatur unter Wasserstoffnormaldruck hydriert. Nach Abfiltrieren vom Katalysator und Einengen des Filtrates erhält man 1,5g (88,4% d.Th.) an 2-N[(2-Amino-4-trifluormethylphenyl)-amino]propionitril.

### C

1,5g (6,5mmol) 2-N[(2-Amino-4-trifluormethylphenyl)-amino]propionitril werden mit 0,8ml (7,3mmol) Triethylamin in 90ml Tetrahydrofuran vorgelegt und bei 0°C tropfenweise mit einer Lösung von 0,75ml (7,3mmol) Oxalsäurehalbethylesterchlorid in 20ml Tetrahydrofuran versetzt. Nach 2h Rühren bei Raumtemperatur wird von den Salzen abgesaugt und das Filtrat eingeengt. Der Rückstand wird in 100ml 1N-Salzsäure mit 100ml Etanol 2h am Rückfluss gekocht. Nach Einengen wird in Essigester/ Wasser verteilt. die organische Phase wird eingeengt und der Rückstand mit Essigester ausgerührt. Man erhält 510mg (44,8% d.Th.) 3-[(6-Trifluormethylchinoxalin-2,3-dion-1-yl)propionitril.

Analog wird hergestellt:
2-[(6 Trifluormethylchinoxalin-2,3-dion-1-yl)acetonitril
2-[(6 Trifluormethylchinoxalin-2,3-dion-1-yl)ethyl]sulfonsäure

### Beispiel 11

### 2-[(6-Trifluormethylchinoxalin-2,3-dion-1-yl)ethyl]-tetrazol

345mg (1,2mmol) 3-[(6-Trifluormethylchinoxalin-2,3-dion-1-yl)propionitril werden zusammen mit 407mg (6,3mmol) Natriumazid und 333mg (6,3mmol) Ammoniumchlorid in 13 ml Dimethylformamid 3h auf 120°C Badtemperatur erwärmt. Nach nochmaliger Zugabe von 203mg (3,2mmol) Natriumazid und 160mg (3,1mmol) Ammoniumchlorid wird nochmals 5h auf 120°C Badtemperatur erwärmt. Nach Verdünnen mit Wasser und Einstellen des pH auf 2 wird mit Essigester extrahiert. Beim Ausschütteln mit gesättigter Kochsalzlösung fällt aus der organischen Phase die gewünschte Verbindung aus. Durch Einengen der organischen Phase und Ausrühren in Essigester erhält man eine weitere Fraktion. Insgesamt erhält man 150mg (37,8% d.Th.) 2-[(6 Trifluormethylchinoxalin-2,3-dion-1-yl)ethyl]-tetrazol vom Schmpkt 279,8°C.

In analoger Weise wird hergestellt:
[(6-Trifluormethylchinoxalin-2,3-dion-1-yl)methyl]-tetrazol

### Beispiel 12

### 2-[(6-Trifluormethylchinoxalin-2,3-dion-1-yl)ethylchlorid

1,3g (4,7mmol) 2-[(6 Trifluormethylchinoxalin-2,3-dion-1-yl)ethylalkohol werden in 20ml Thionylchlorid 4h bei Raumtemperatur gerührt. Nach Einengen und Nachdestillieren mit Toluol erhält man 1,34g (97% d.Th.) 2-[(6 Trifluormethylchinoxalin-2,3-dion-1-yl)ethylchlorid.

### Beispiel 13

### N-[(6-Trifluormethylchinoxalin-2,3-dion-1-yl)ethylimidazol

392mg (1mmol) 2-[(6 Trifluormethylchinoxalin-2,3-dion-1-yl)ethylchlorid werden mit 150mg (2,2mmol) Imidazol 3h auf 150°C Badtemperatur erwärmt. Beim Verteilen in 10ml Essigester und 10ml Wasser wird die organische Phase eingeengt und der Rückstand über Kieselgel mit Methanol: Butanol: Wasser: Ammoniak= 75: 25: 17: 3 als Elutionsmittel chromatographiert. Man erhält 54mg (17% d.Th.) 2-[(6 Trifluormethylchinoxalin-2,3-dion-1-yl)ethylimidazol vom Schmpkt >250°C.

Analog wird hergestellt:
2-[(6 Trifluormethylchinoxalin-2,3-dion-1-yl)ethylmorpholin (als Harz).

### Beispiel 14

### 6-Trifluormethyl-7-nitro-1-(1-methoxyeth-2-yl)-1,2,3,4-tetrahydro-2,3-dioxo-chinoxalin.

100mg 6-Trifluormethyl-1-(1-methoxyeth-2-yl)-1,2,3,4-tetrahydro-2,3-dioxo-chinoxalin werden in 1ml konz. Schwefelsäure aufgeschlämmt, bei 4°C mit 0,1ml einer Mischung aus konz. Schwefelsäure: konz. Salpetersäure=1:1 versetzt und 1h bei 4°C gerührt. Danach ist alles in Lösung gegangen. Anschliessend wird auf Eis gegeben und der ausgefallene Niederschlag abgesaugt. Man erhält 59mg (50% d.Th.) an 6-Trifluormethyl-7-Nitro-1-(1-methoxyeth-2-yl)-1,2,3,4-tetrahydro-2,3-dioxo-chinoxalin.

### Beispiel 15

### 6-Trifluormethyl-7-jod-1-(1-methoxyeth-2-yl)-1,2,3,4-tetrahydro-2,3-dioxo-chinoxalin.

288mg 6-Trifluormethyl-1-(1-methoxyeth-2-yl-1,2,3,4-tetrahydro-2,3-dioxo-chinoxalin werden in 5ml Eisessig mit o,o5ml Wasser, 0,012ml konz. Schwefelsäure, 34,4 mg Jodsäure und 88,4mg Jod versetzt und 4h auf 80°C erwärmt. Nach Einengen wird in Wasser aufgenommen alkalisch gestellt und mit Methylenchlorid extrahiert. Die organische Phase wird getrocknet, filtriert und eingeengt und der Rückstand über Kieselgel mit Toluol: Eisessig: Wasser= 10: 10: 1 als Elutionsmittel chromatographiert. Man erhält 40mg 6-Trifluormethyl-7-jod-1-(1-methoxyeth-2-yl-1,2,3,4-tetrahydro-2,3-dioxo-chinoxalin. Der Rest ist Ausgangsmaterial, das nochmals in die Reaktion eingesetzt werden kann.

### Beispiel 16

### N-[(6 Cyanochinoxalin-2,3-dion)-1-yl]ethanphosphonsäurediethylester

### A

2,77g (15,24mmol 4-Chlor-3-nitrobenzonitril werden mit 10,5g (38mmol) 1-Aminoethanphosphonsäurediethylester 16h bei 30°C gerührt. Die Reaktionsmischung wird dann über Kieselgel mit Methylenchlorid:Etanol=95:5 als Elutionsmittel chromatographiert. Man erhält 3,86g (80% d.Th.) 1-[N-(2-Nitro-4-cyanophenyl)amino]ethanphosphonsäureedietylester.

### B.

3,1g N-(2-Nitro-4-cyanophenyl)aminoethanphosphonsäureedietylester werden in 55ml Tetrahydrofuran mit 3ml Triethylamin gelöst und bei 0°C tropfenweise mit einer Lösung von 2,35ml Oxalsäurehalbethylesterchlorid in 16ml Tetrahydrofuran versetzt. Nach dreitägigem Rühren bei Raumtemperatur wird mit 300ml Essigester verdünnt und nacheinander mit Wasser und konz. Kochsalzlösung gewaschen. Die organische Phase wird getrocknet, filtriert und eingeengt. Man erhält 5,43g rohes N-(Diethylphosphonyleth-1-yl)-N-(2-nitro-4-cyanophenyl)oxalsäurehalbethyl-esteramid, das ohne weitere Reinigung weiterumgesetzt wird.

### C.

1,25g N-(Diethylphosphonyleth-1-yl)-N-(2-nitro-4-cyanophenyl)oxalsäurehalbethylesteramid werden in 60ml Eisessig mit 12,9g (230mmol) Eisenpulver versetzt und 60 min auf 90°C erwärmt. Nach Abdekantieren vom nicht umgesetzten Eisen und Filtration über Kieselgur wird das Filtrat eingeengt, in Essigester aufgenommen und mit Wasser mehrfach gewaschen. Die organische Phase wird getrocknet, filtriert und eingeengt. der Rückstand wird über Kieselgel mit Toluol:Etanol=8:2 als Elutionsmittel chromatographiert. Man erhält 1,43g (32,9% d.Th.) an N-[(6 Cyanochinoxalin-2,3-dion)-1-yl]ethanphosphonsäurediethylester.

### Beispiel 17

### N-[(6 Cyanochinoxalin-2,3-dion)-1-yl]ethanphosphonsäure

600mg N-[(6 Cyanochinoxalin-2,3-dion)-1-yl]ethanphosphonsäure-diethylester werden in 20ml Methylenchlorid gelöst und langsam mit 2ml (13,4mmol) Trimethylsilyljodid tropfenweise versetzt. Nach 4h Rühren bei Raumtemperatur ist eine braune Lösung entstanden. Man schüttelt mit Wasser aus und saugt die dabei entstandenen Fällungen ab. Die vereinigten Kristallisate werden aus Ethanol/ Wasser umkristallisiert. Man erhält 300mg (83% d.Th.) N-[(6-Cyanochinoxalin-2,3-dion)-1-yl]ethanphosphonsäure vom Schmelzpunkt 280°C.

### Beispiel 18

### N-[6-Trifluormethyl-7-amino-1,2,3,4-tetrahydro-2,3-dioxo-chinoxalin-1-yl]-methanphosphonsäurediethylester.

600 mg N-[6-Trifluormethyl-7-nitro-1,2,3,4-tetrahydro-2,3-dioxo-chinoxalin-1yl]-methanphosphonsäureester werden in 30ml Ethanol gelöst und zusammen mit 100mg Pd/C **(10%)** 1h bei Wasserstoffnormaldruck und Raumtemperatur hydriert. Nach Absaugen vom Katalysator wird der Katalysator nochmals mit Ethanol ausgekocht, das gesammelte Filtrat eingeengt und man erhält 590 mg N-[6-Trifluormethyl-7-amino-1,2,3,4-tetrahydro-2,3-dioxo-chinoxalin-1yl]-ethanphosphonsäurediethylester.

Analog werden hergestellt:
N-[6-Trifluormethyl-7-amino-1,2,3,4-tetrahydro-2,3-dioxo-chinoxalin-1yl]-methanphosphonsäurediethylester.
N-[6-Trifluormethyl-7-amino-1,2,3,4-tetrahydro-2,3-dioxochinoxalin-1-yl]-essgsäureethylester
6-Trifluormethyl-7-amino-1-(1-methoxyeth-2-yl)-1,2,3,4-tetrahydro-2,3-dioxochinoxalin

### Beispiel 19

### N-[6-Trifluormethyl-7-nitro-1,2,3,4-tetrahydro-2,3-dioxochinoxalin-1-yl]-essigsäureethylester

A.)
   (1,1mmol) 2-[(6 Trifluormethylchinoxalin-2,3-dion-1-yl)acetonitril werden in 10ml konzentrierter Salzsäure 4h am Rückfluss gekocht. Es wird das ausgefallene Produkt abgesaugt. Man erhält 188mg 2-[(6 Trifluormethylchinoxalin-2,3-dion-1-yl)essigsäure.
B.) 150mg (0,52mmol) 2-[(6 Trifluormethylchinoxalin-2,3-dion-1-yl)essigsäure werden in 7ml ethanolischer Salzsäure 2h am Rückfluss gekocht. Danach wird eingeengt, der Rückstand in 4ml Methylenchlorid und 1ml Acetonitril gelöst, mit 120mg Nitroniumterafluoroborat versetzt und zwei Stunden bei Raumtemperatur gerührt. Anschliessend wird mit Wasser geschüttelt, getrocknet, flitriert und eingeengt. Man erhält N-[6-Trifluormethyl-7-nitro-1,2,3,4-tetrahydro-2,3-dioxochinoxalin-1-yl]-essigsäureethylester.

### Beispiel 20

### 6-Trifluormethyl-7-jod-1-(1-methoxyeth-2-yl-1,2,3,4-tetrahydro-2,3-dioxo-chinoxalin.

90mg 6-Trifluormethyl-7-amino-1-(1-methoxyeth-2-yl-1,2,3,4-tetrahydro-2,3-dioxo-chinoxalin werden in 8ml ethanolischer Salzsäure aufgenommen und eingeengt und das Hydrochlorid in 6ml Dimethylformamid und 3ml Methylenjodid aufgenommen und bei 80°C Badtemperatur mit 0,08ml i-Amylnitrit versetzt. Nach 3h Rühren bei dieser Temperatur wird der Ansatz am Vakuum eingeengt. Man erhält 105mg 6-Trifluormethyl-7-jod-1-(1-methoxyeth-2-yl-1,2,3,4-tetrahydro-2,3-dioxo-chinoxalin.

In analoger Weise werden hergestellt:
N-[6-Trifluormethyl-7-jod-1,2,3,4-tetrahydro-2,3-dioxo-chinoxalin-1-yl]-methanphosphonsäurediethylester.
N-[6-Trifluormethyl-7-jod-1,2,3,4-tetrahydro-2,3-dioxo-chinoxalin-1-yl]-essigsäureethylester.

### Beispiel 21

### 1-[(6-Trifluormethyl-7-[piperidin-1-yl]chinoxalin-2,3-dion)-1yl]-ethanphosphonsäure-diethylester;

Bei 0°C wird zu einer Aufschlemmung von 100mg [(6-Trifluormethyl-7-amino-1-yl]chinoxalin-2,3-dion)-1yl]-methanphosphonsäure-diethylester und 30mg Natiumborhydridtablettenstücken in 3 ml Tetrahydrofuran eine Lösung von 0,15ml einer 25%igen wässrigen Lösung von Glutardialdehyd und 0,45ml 3-M Schwefelsäure in 3ml Tetrahydrofuran:Methanol=2:3 getropft. Nach Abklingen der Reaktion werden nocheinmal 30mgNatiumborhydridtablettenstücken nachgeworfen und anschliessend 1h bei Raumtemperatur gerührt. Dann wird mit Natronlauge neutral gestellt und mit Essigester ausgeschüttelt. Die Essigesterphase wird getrocknet, filtriert und eingeengt. Nach Chromatographie über Kieselgel mit Toluol:Eisessig:Wasser=10:10:1 erhält man 60mg 1-[(6-Trifluormethyl-7-[piperidin-1-yl]chinoxalin-2,3-dion)-1yl]-ethanphosphonsäure-diethylester.

In analoger Weise werden hergestellt:
1-[(6-Trifluormethyl-7-[piperidin-1-yl]chinoxalin-2,3-dion)-1yl]-essigsäurethylester;

### Beispiel 22

### 1-[(6-Trifluormethyl-7-propylamino-]chinoxalin-2,3-dion)-1yl]-ethanphosphonsäure-diethylester;

Bei 4°C wird zu einer Aufschlemmung von 80mg [(6-Trifluormethyl-7-amino-1-yl]chinoxalin-2,3-dion)-1yl]-methanphosphonsäure-diethylester in 3ml Tetrahydrofuran eine Lösung von 0,02ml destilliertem Propanal und 0,2ml 3-M Schwefelsäure in 2ml Tetrahydrofuran getropft. Zu dieser gerührten Mischung werden 20mg Natiumborhydridtablettenstücken gegeben. Nach Abklingen der Reaktion werden nocheinmal 10mgNatiumborhydridtablettenstücken nachgeworfen und anschliessend 1h bei Raumtemperatur gerührt. Dann wird mit Natronlauge neutral gestellt und mit Essigester ausgeschüttelt. Die Essigesterphase wird getrocknet, filtriert und eingeengt. Nach Chromatographie über Kieselgel mit Toluol:Eisessig:Wasser=10:10:1 erhält man 40mg 1-[(6-Trifluormethyl-7-propylamino-chinoxalin-2,3-dion)-1yl]-ethanphosphonsäure-diethylester.

### Beispiel 23

### 1-[(6-Trifluormethyl-7acetylamino-]chinoxalin-2,3-dion)-1yl]-ethanphosphonsäure-diethylester

80mg 1-[(6-Trifluormethyl-7-amino-]chinoxalin-2,3-dion)-1yl]-ethanphosphonsäure-diethylester werden in 8ml Essigsäure gelöst und mit 150mg Acetanhydrid versetzt und 3h bei Raumtemperatur gerührt. Nach Einengen erhält man 50mg 1-[(6-Trifluormethyl-7-acetylamino-]chinoxalin-2,3-dion)-1yl]-ethanphosphonsäure-diethylester.

## Patentansprüche

1. Verbindungen der Formel I 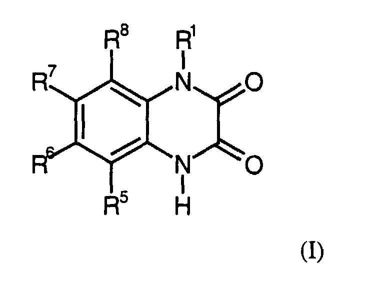 worin
R¹ -(CH₂)ₙ-CR²H-(CH₂)ₘ-Z und
R⁵, R⁶, R⁷ und R⁸ gleich oder verschieden sind und Wasserstoff, CF₃ oder NR⁹R¹⁰ bedeuten,
wobei
R² Wasserstoff oder -(CH₂)_{q}-R³ ,
R³ Wasserstoff, Hydroxy, C₁₋₆-Alkoxy oder NR¹⁵R¹⁶,
n, m und q jeweils 0, 1, 2 oder 3
Z CO₂R²³,
R²³ Wasserstoff oder C₁₋₄-Alkyl,
R⁹ und R¹⁰ gleich oder verschieden sind und Wasserstoff, Phenyl oder C₁₋₆-Alkyl, das gegebenenfalls mit C₁₋₄-Alkoxy oder einer gegebenenfalls mit C₁₋₄-Alkyl mono- oder disubstituierter Aminogruppe substituiert sein kann, oder gemeinsam mit dem Stickstoffatom einen 5 - 7gliedrigen gesättigten Heterocyclus bilden, der ein weiteres N-, S- oder O-Atom enthalten und substituiert sein kann oder einen ungesättigten 5-gliedrigen Heterocyclus bilden, der 1 - 3 N-Atome enthalten und substituiert sein kann,
R¹⁵ und R¹⁶ gleich oder verschieden sind und Wasserstoff, C₁₋₄-Alkyl, Phenyl oder gemeinsam mit dem Stickstoffatom einen 5 - 7gliedrigen gesättigten Heterocyclus bilden, der ein weiteres Sauerstoff-, Schwefel- oder Stickstoffatom enthalten und substituiert sein kann oder einen ungesättigten 5gliedrigen Heterocyclus bilden, der 1 - 3 N-Atome enthalten und substituiert sein kann,
sowie deren Isomeren und Salze, die in Position 5 - 8 zweifach substituiert sind.

2. 1-[6-Trifluormethyl-7-[piperidin-1-yl]chinoxalin-2,3-dion)-1-yl]essigsäure
N-[6-Trifluormethyl-7-amino-1,2,3,4-tetrahydro-2,3-dioxochinoxalin-1-yl]essigsäure

3. Verbindungen nach Anspruch 1, worin NR⁹R¹⁰ Piperidin, Pyrrolidin, Morpholin, Thiomorpholin, Hexyhydroazepin oder Piperazin ist und der Heterocyclus 1-3fach mit C₁₋₄-Alkyl oder Phenyl substituiert sein kann.

4. Arzneimittel enthaltend eine Verbindung nach Anspruch 1 und einen oder mehrere pharmazeutisch geeignete Träger- und Hilfsstoffe.

5. Verfahren zur Herstellung der Verbindungen der Formel I, dadurch gekennzeichnet, daß man
a) eine Verbindung der Formel II 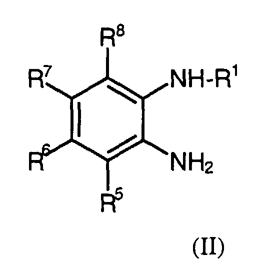 worin R¹ bis R⁸ die obige Bedeutung haben, mit Oxalsäure oder reaktiven Oxalsäurederivaten cyclisiert oder
b) eine Verbindung der Formel III 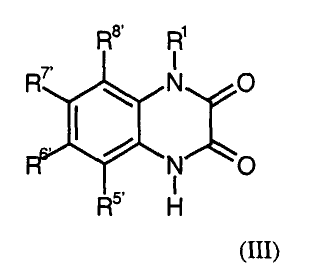 worin R¹ die obige Bedeutung hat und einer der Substituenten R^{5'},R^{6'}, R^{7'} oder R^{8'} eine Fluchtgruppe darstellt, nucleophil substituiert und gewünschtenfalls anschließend die Estergruppe verseift oder die Säuregruppe verestert oder amidiert oder die Nitrogruppe reduziert zur Aminogruppe oder die Aminogruppe alkyliert oder acyliert oder die Aminogruppe gegen Halogen oder Cyano austauscht oder eine Nitrogruppe oder Halogen einführt oder eine Etherspaltung vornimmt oder den Alkohol in Halogenid überführt oder dieses Halogen
nukleophil substituiert oder ein Nitril in das Tetrazol überführt oder die Isomeren trennt oder die Salze bildet.

6. Verwendung einer Verbindung nach Anspruch 1 zur Herstellung eines Arzneimittels zur Behandlung einer durch Hyperaktivität excitatorischer Aminosäuren ausgelösten Erkrankung.

7. Verwendung einer Verbindung nach Anspruch 1 zur Herstellung eines Arzneimittels zur Behandlung neurologischer und psychiatrischer Erkrankungen.

8. Verwendung nach Anspruch 9 zur funktionellen und präventiven Behandlung neurodegenerativer Erkrankungen.

9. Verwendung nach Anspruch 10 zur Behandlung von Stroke.
